# EUROPEAN PATENT SPECIFICATION

(11) **EP 4 339 185 B1**
(45) Date of publication and mention of the grant of the patent: **23.07.2025**
(21) Application number: 22306362.9
(22) Date of filing: 16.09.2022
(51) Int. Cl.: C07C 67/333, C07C 67/54, C07C 69/54

(54) **METHOD AND INSTALLATION FOR THE PRODUCTION OF A MONOMER BY DEPOLYMERIZATION OF THE CORRESPONDING POLYMER**
VERFAHREN UND ANLAGE ZUR HERSTELLUNG EINES MONOMERS DURCH DEPOLYMERISIERUNG DES ENTSPRECHENDEN POLYMERS
PROCEDE ET INSTALLATION DE PRODUCTION D'UN MONOMERE PAR DEPOLYMERISATION DU POLYMERE CORRESPONDANT

(43) Date of publication of application: 20.03.2024
(73) Proprietor: ARKEMA FRANCE, 92800 Puteaux (FR); Japan Steel Works Europe GmbH, 40589 Düsseldorf (DE); Trinseo Europe GmbH, 8810 Horgen (CH)
(72) Inventor: DUBOIS, Jean-Luc, 92700 COLOMBES (FR); TOJO, Makoto, 40217 DÜSSELDORF (DE); VAN DER HEIJDEN, Simon, 3402SE IJSSELSTEIN (NL)
(74) Representative: Lavoix

(56) References cited:
- CN-B- 109 761 814
- JP-A- H0 789 900
- US-A- 5 663 420
- C.B. GODIYA, ET AL.: "Depolymerisation of waste poly(methyl methacrylate) scraps and purification of depolymerised products", JOURNAL OF ENVIRONMENTAL MANAGEMENT, vol. 231, 13 November 2018 (2018-11-13), Elsevier, Oxford, GB, pages 1012 - 1020, XP055878591, ISSN: 0301-4797, DOI: 10.1016/j.jenvman.2018.10.116

## Description

The project leading to this patent application has received funding from the European Union's Horizon 2020 research and innovation program under grant agreement No 820687.

The invention relates to the field of depolymerization for recovering a monomer from a feed material containing a polymer from which the monomer is a constituent.

In particular, the invention relates to the field of depolymerization of polymethyl methacrylate (PMMA) for recovering methyl methacrylate (MMA).

Recycled MMA is obtained for example by pyrolyzing solid PMMA wastes in a pyrolysis reactor, in particular a pyrolysis reactor provided as a heated screw extruder, such as to obtain a gas stream including gaseous MMA, removing unavoidable solid and/or liquid impurities included in the gas stream and condensing the gas stream to obtain liquid MMA.

The liquid MMA obtained from depolymerization of the PMMA is sometimes named "crude MMA".

Besides, some PMMA wastes are liquid products. These are typically syrups which are produced to make cast PMMA sheets and which cannot be used, for example because of quality defaults or change of product demands or failure of equipment, and which evolved too much during storage to guarantee proper product quality if used.

In view of improving the recycling efficiency, it would be advantageous to be able to process the solid and/or liquid impurities removed from the gas stream and/or liquid PMMA wastes.

CN109761814B discloses a method of producing MMA. US5663420A and JPH0789900A disclose methods of recovering a monomer from a polymer. C.B. GODIYA, ET AL.: "'Depolymerisation of waste poly (methyl methacrylate) scraps and purification of depolymerised products", JOURNAL OF ENVIRONMENTAL MANAGEMENT, vol. 231, 13 November 2018, discloses experiments on pyrolyzing PMMA.

One of the aims of the invention is to propose a process of producing a monomer that allows obtaining a monomer solution with a satisfactory purity.

To the end, the invention proposes a method for the production of a monomer as defined in claim 1.

A syrup means a solution of polymer and/or oligomers in the monomer, e.g. a solution of PMMA or MMA oligomers in MMA. Such syrup is obtained for example by a prepolymerization of the monomer, e.g. prepolymerization of MMA, as commonly done for the production of cast PMMA sheets. Alternatively, such a syrup is obtained for example by dissolution of the polymer in the monomer, e.g. the dissolution of PMMA in MMA. In the latter case it is preferable to use lower molecular weight polymer grades, e.g. lower molecular weight PMMA grades which might have a better solubility.

Solid and liquid impurities separated from the gas stream generated in the pyrolysis reactor may include depolymerization residues mixed with some monomer, e.g. MMA. The solid impurities include for example fine dusts of pigments and their degradation products, contaminating polymers, reinforcing agents such as glass and carbon fibers, wood residues, monomers and oligomers from other polymers as well as their degradation products when they are non soluble in the liquid medium. The liquid impurities may include the monomer, its oligomers, but also the polymer fraction which is soluble in the monomer, all the degradation products from the polymer itself and its contaminants. To some extent, the liquid impurities may comprise some water, hydrocarbons such as for example degradation products from the polyethylene film that covers PMMA sheets, aromatics such as toluene, xylenes and heavy aromatics generated by degradation of the polymers and contaminants but also generated from the decomposition of additives such as shock resistant additives. In practice, a major part of the liquid and solid impurities are constituted of the monomer, dimers and trimers and degradation products, but also combination of monomer and comonomers and their degradation products, as well as solutions of polymers and oligomers.

Optional features of the production method are defined in claims 2 - 14.

The invention also relates to an installation for the production of a monomer as defined in claim 15.

The invention and its advantages will be better understood upon reading the following description that is given solely by way of non-limiting example and given with reference to the appended drawings, in which:
- Figure 1 is schematic diagram illustrating a depolymerization installation for the production of a monomer solution from a feed material containing a polymer from which the monomer is a constituent;
- Figure 2 is schematic diagram illustrating a pyrolysis reactor provided as a heated screw extruder.

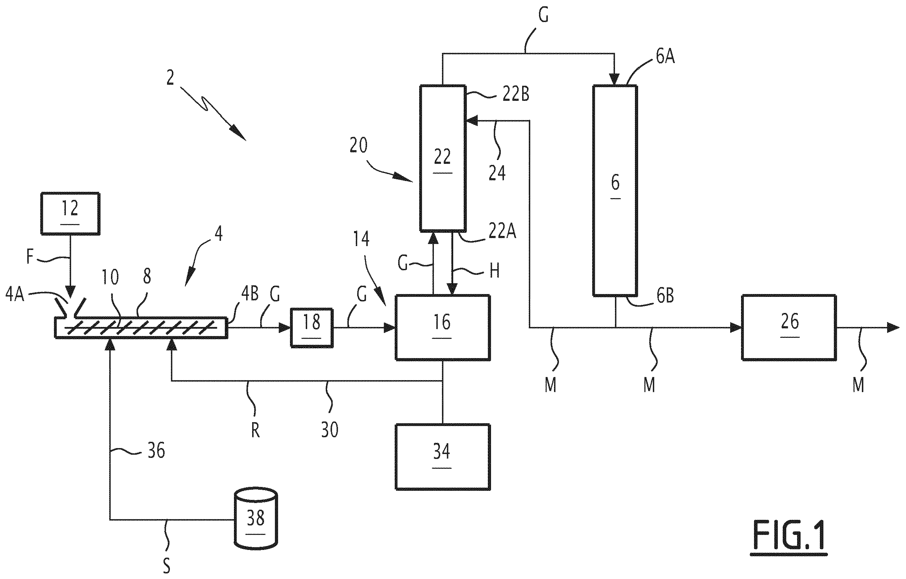

The depolymerization installation 2 of Figure 1 is configured for the production of a monomer from a feed material F containing a polymer from which the monomer is a constituent.

The depolymerization installation 2 comprises a pyrolysis reactor 4 configured to receive the feed material and to generate a gas stream G containing the monomer in gaseous state and a condenser 6 configured for condensing the gas stream G such as to obtain a monomer solution M containing the monomer in liquid state.

In the following, the monomer solution M resulting from the condensing of the gas stream G is also referred to as the "crude monomer solution" or "crude monomer" or "liquid monomer".

The pyrolysis reactor 4 is advantageously a heated screw extruder comprising a heated barrel 8 and at least one screw 10 arranged inside the barrel 8 for moving the feed material through the barrel 8 with heating the feed material.

The depolymerization installation 2 comprises preferably a feeder 12 configured for feeding the feed material F to an inlet 4A of the pyrolysis reactor 4.

The depolymerization installation 2 comprises optionally a separation unit 14 fluidly connected in series between an outlet 4B of the pyrolysis reactor 4 providing the gas stream G and the inlet of the condenser 6, the separation unit 14 being configured for separating solid and/or liquid impurities from the gas stream G before feeding the gas stream G to the condenser 6.

The separation unit 14 comprises for example a main separator 16 configured for diminishing the linear velocity of the gas stream G to promote the separation and deposit of solid impurities and/or for demisting the gas stream G to promote the deposition of mist droplets of liquid impurities and/or for washing the gas stream with a washing liquid to promote deposition of liquid impurities.

The diminution of the linear velocity of the gas stream G is obtained for example by a progressive increasing of the area of the cross-section of an internal flow passage of the main separator 16 through which the gas stream G passes in the main separator 16.

Preferably, the main separator 16 is configured such that the internal flow passage of the main separator 16 does not follow a straight line. Such a configuration promotes separation and deposition of impurities.

The demisting of the gas stream G is obtained e.g. by providing one or several demisting grids across the internal flow passage of the main separator 16.

The washing of the gas stream G is obtained e.g. by injection of water and/or injection of liquid monomer inside the internal flow passage of the main separator 16.

The separation unit 14 comprises optionally an auxiliary separator 18 arranged upstream the main separator 16. The gas stream G generated by the pyrolysis reactor 4 flows through the auxiliary separator 18 before entering the main separator 16.

The auxiliary separator 18 is for example configured for removing solid impurities from the hot gas stream G by inertia.

The auxiliary separator 18 is for example configured with an internal flow passage having a change of cross section promoting a decrease of linear velocity and/or a change of direction that are located above a collecting pot such that the solid particles slowed down by the decrease of linear velocity and/or driven by inertia tend to hit walls of the internal flow passage and to fall down in the collecting pot.

Advantageously, the depolymerization installation 2 comprises a gas cleaning unit 20 connected in series between the separation unit 14 and the condenser 6 and configured for removing heavies from the gas stream G before the gas stream enters the condenser 6.

The gas cleaning unit 20 is arranged such that the gas stream G exiting the separation unit 14 passes via the gas cleaning unit 20 before entering the condenser 6.

The gas cleaning unit 20 comprises a transfer column 22 extending upwardly between a lower end 22A and an upper end 22B. The lower end 22A is fluidly connected to the separation unit 14, in particular to the main separator 16, for receiving the gas stream G exiting the separation unit 14. The upper end 22B is fluidly connected to an inlet 6A of the condenser 6.

The gas cleaning unit 20 comprises a reinjection loop 24 configured for reinjecting a fraction of the monomer solution collected at an outlet 6B of the condenser 6 into a top portion of the transfer column 22, in particular at the top end 22B of the transfer column 22.

The transfer column 22 is provided with internals. The internals comprise for example baffles, structure packing, gauze packing and/or random packing, e.g. random packing including saddles and/or rings, in particular Pall rings.

The internals are configured such that, in operation, the gas stream flows upwardly in the transfer column 22, the temperature of the gas stream diminishing along the transfer column 22, and the monomer solution injected in the transfer column 22 flows downwardly inside the transfer column 22 by gravity, heavy contaminants H contained in the gas stream condense on the internals and flow back in liquid state down the transfer column 22 and into the separation unit 14, in particular into the main separator 16.

The depolymerization installation 2 optionally comprises a purification unit 26 configured for purifying the monomer solution such as to obtain a purified monomer solution M containing less impurities.

The purification unit 26 comprises for example an evaporation-condensation unit configured for successively evaporating and condensing the monomer solution with removing heavies in the evaporator, one or several distillation columns, a thin-film evaporator, a short-path distillation unit and/or a crystallization unit comprising one or several crystallizers configured for performing a fractional crystallization, or any other appropriate technology or combination of technologies for purifying the monomer solution.

The installation 2 is configured for feeding the pyrolysis reactor 4 with solid and/or liquid impurities R separated from the gas stream G generated in the depolymerization installation 2 and/or from a gas stream generated in another depolymerization installation and/or with a syrup S containing the polymer and/or the monomer and/or oligomers of the monomer.

The depolymerization installation 2 comprises for example a reinjection line 30 configured for feeding the pyrolysis reactor 4 with solid and/or liquid impurities R separated from the gas stream generated in the depolymerization installation 2 and/or from a gas stream generated in another depolymerization installation

For example, the reinjection line 30 connects the separation unit 14 to the pyrolysis reactor 4 for injecting solid and/or liquid impurities R separated from the gas stream G in the separation unit 14 into the pyrolysis reactor 4 and/or connects another separation unit 34 of another depolymerization installation to the pyrolysis reactor 4 for injecting solid and/or liquid impurities separated from the gas stream in this other separation unit 34 into the pyrolysis reactor 4.

In particular, the reinjection line 30 connects the main separator 16 of the separation unit 14 to the pyrolysis reactor 4 and/or connects a main separator of the other separation unit 34 to the pyrolysis reactor 4.

The depolymerization installation 2 comprises for example an injection line 36 configured for feeding the pyrolysis reactor 4 with the syrup S, for example from a syrup source 38, such as a syrup reservoir.

The barrel 8 is preferably configured for heating the material contained in the barrel 8 according to a temperature profile, i.e. a temperature that varies along the barrel 8.

Preferably, the pyrolysis reactor 4 is configured to generate a temperature profile that progressively increases and then progressively decreases from the inlet of the pyrolysis reactor 4 receiving the feed material to the outlet of the pyrolysis reactor 4 delivering the gas stream.

Typically, the temperature profile is such that an inlet temperature is higher than 100 °C, a maximum temperature is higher than 250°C, in particular higher than 350°C, more in particular higher than 400°C, and an outlet temperature is higher than 100°C.

Preferably, the solid and liquid impurities are fed to the pyrolysis reactor 4 in a section of the pyrolysis reactor 4 that is preferably proximate the outlet 4B of the pyrolysis reactor 4, and in particular closer to the outlet 4B than the inlet 4A, i.e. the second half of the length of the barrel 8 of the pyrolysis reactor 4.

The section of the pyrolysis reactor 4 in which the solid and liquid impurities R are fed to the pyrolysis reactor 4 is preferably at a temperature below 300°C, preferably below 250°C, still preferably below 200°C.

The section of the pyrolysis reactor 4 in which the solid and liquid impurities R are fed to the pyrolysis reactor 4 is preferably at a temperature above 100°C or a temperature above the boiling temperature of the monomer.

Preferably the syrup is fed to a section of the pyrolysis reactor 4 that is proximate the inlet 4A of the pyrolysis reactor 4, and in particular closer to the inlet 4A than the outlet 4B, i.e. in the first half of the length of the barrel 8 of the pyrolysis reactor 4.

The section of the pyrolysis reactor 4 in which the syrup S is fed to the pyrolysis reactor 4 is preferably at the temperature below 450 °C, more preferably below 350 °C, more preferably below 300°C, preferably below 250°C.

When both solid and/or liquid impurities R and syrup S are injected into the pyrolysis reactor 4, they are preferably injected in separate sections of the pyrolysis reactor 4. The point of injection of the solid and/or liquid impurities R into the pyrolysis reactor 4 is separate and distinct from the point of injection of the syrup S into the pyrolysis reactor 4.

Preferably, feed material is injected in the pyrolysis reactor 4 such as to create a solid plug between the point of injection of the syrup S into the pyrolysis reactor 4 and the inlet 4A of the pyrolysis reactor 4 receiving the feed material F. In particular, the feed material F comprises or consists in solid material.

The presence of a plug in the pyrolysis reactor 4 prevents gaseous MMA and other decomposition products, solid and/or liquid impurities and/or syrup to flow backwards in the pyrolysis reactor 4, towards the inlet 4A receiving the feed material F.

Preferably, the pyrolysis reactor 4 is configured such that the temperature in a section of the pyrolysis reactor 4 located between a point of injection of the syrup S and the point of injection of solid and/or liquid impurities R is above 250 °C, preferably above 350 °C, in particular above 400 °C.

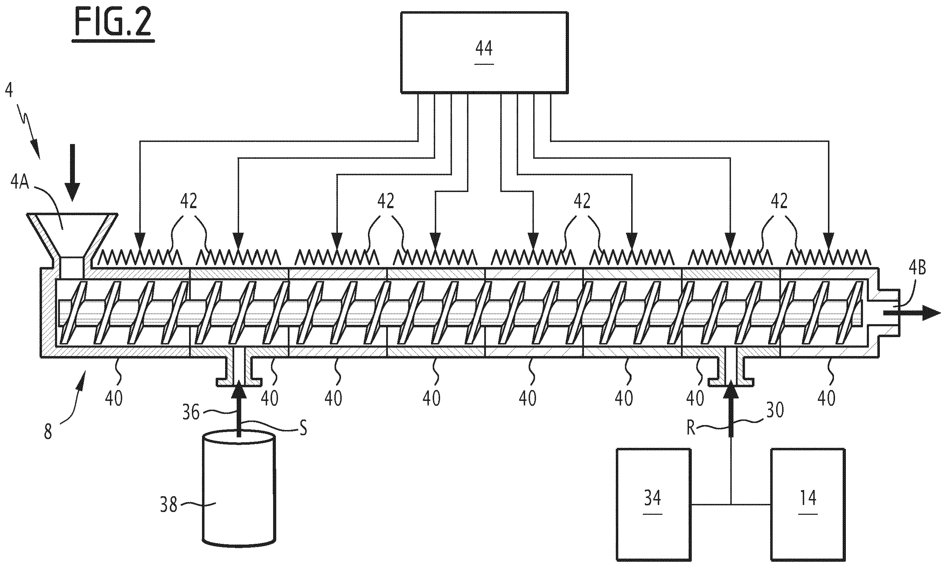

As illustrated on Figure 2, the barrel 8 of the pyrolysis reactor 4 is for example defined by a series of barrel sections 40 (also called "barrel cylinders") connected in series, each barrel section 40 being provided with a respective heating device 42 for heating this particular barrel section 40. Each barrel section 40 (or barrel cylinder) defines a portion of length of the barrel 8.

The pyrolysis reactor 4 comprises for example an electrical control unit 44 configured for controlling the heating devices 42 of the barrel sections 40 individually such as to generate the desired temperature profile in the barrel 8.

Preferably, the inlet of the pyrolysis reactor 4 receiving the feed material is located on the first barrel section 40 of the series of barrel sections 40 and/or the outlet of the pyrolysis reactor 4 delivering the gas stream G is located on the ultimate barrel section 40 of the series of barrel sections 40.

Preferably, solid and/or liquid impurities R are fed in the antepenultimate barrel section 40, the penultimate barrel section 40 and/or the ultimate barrel section 40 of the series of barrel sections 40.

As illustrated on Figure 2, solid and/or liquid impurities R are fed in the penultimate barrel section 40 of the series of barrel sections 40

As illustrated on Figure 2, the syrup is fed in the second barrel section 40 of the series of barrel sections 40.

This is illustrative only. In practice, the barrel section 40 in which the syrup is injected is chosen as a function of the operative conditions.

The syrup is injected for example in the second barrel section, the third barrel section, the fourth barrel section, the fifth barrel section or the sixth barrel section.

In operation, the depolymerization installation 2 is configured for implementing a depolymerization method comprising the steps of:
- pyrolyzing the feed material F in the pyrolysis reactor 4 so as to generate the gas stream G, the feed material F travelling from the an inlet 4A of the pyrolysis reactor 4 to an outlet 4B of the pyrolysis reactor with being subjected to heat such as to generate the gas stream G at the outlet of the pyrolysis reactor 4; and
- condensing the monomer contained in the gas stream G in the condenser 6 such as to obtain the liquid monomer M.

The production method further comprises feeding the pyrolysis reactor 4 with solid and/or liquid impurities R separated from the gas stream G generated in the depolymerization installation 2 and/or a gas stream generated in another depolymerization installation and/or with a syrup S containing the polymer and/or the monomer and/or oligomers of the monomer.

When applicable, the solid and/or liquid impurities R are preferably fed to the pyrolysis reactor 4 in a section of the pyrolysis reactor 4 that is proximate the outlet 4B of the pyrolysis reactor 4, and in particular closer to the outlet 4B than the inlet 4A.

The section of the pyrolysis reactor 4 in which the solid and liquid impurities R are fed to the pyrolysis reactor 4 is preferably at a temperature below 300°C, preferably below 250°C, still preferably below 200°C.

The section of the pyrolysis reactor 4 in which the solid and liquid impurities R are fed to the pyrolysis reactor 4 is preferably at a temperature above the boiling temperature of the monomer and/or a temperature above 100°C.

When applicable, the syrup S is preferably fed to a section of the pyrolysis reactor 4 that is proximate the inlet 4A of the pyrolysis reactor 4, and in particular closer to the inlet 4A than the outlet 4B.

The section of the pyrolysis reactor 4 in which the syrup S is fed to the pyrolysis reactor 4 is at the temperature below 450 °C, more preferably below 350 °C, more preferably below 300°C, preferably below 250°C.

Preferably, the depolymerization method comprises creating a solid plug between the point of injection of the syrup S into the pyrolysis reactor 4 and the inlet 4A of the pyrolysis reactor 4 receiving the feed material F.

The feed material F is preferably fed to the first barrel section 40 of a series of barrel sections 40 forming the barrel 8.

Preferably, solid and/or liquid impurities R are fed in the antepenultimate barrel section 40, the penultimate barrel section 40 and/or the ultimate barrel section 40 of the series of barrel sections 40.

The syrup S is for example fed in a barrel section comprised between the second barrel section 40 and the sixth barrel section 40 of the series of barrel sections 40.

Preferably, the temperature of the gas stream G at the outlet of the main separator 16 and entrance of the transfer column 22 is maintained at least at the boiling point of the monomer, in particular at least at the boiling point of the monomer + 10°C, in particular at least at the boiling point of the monomer + 20°C, in particular at least at the boiling point of the monomer + 30°C, more in particular at least at the boiling point of the monomer + 40°C, even more in particular at least at the boiling point of the monomer + 50°C.

Preferably the gas stream G at the outlet of the transfer column 22 is maintained between the boiling point of the monomer and the boiling point of the monomer + 50°C, in particular between the boiling point of the monomer and the boiling point of the monomer + 40°C, in particular between the boiling point of the monomer and the boiling point of the monomer + 30°C, in particular between the boiling point of the monomer and the boiling point of the monomer + 20°C, in particular between the boiling point of the monomer and the boiling point of the monomer + 10°C.

The monomer if for example methyl methacrylate (MMA), the polymer being poly (methyl methacrylate) (PMMA).

The provision of the separation step performed in the separation unit 14 and the cleaning step performed in the cleaning unit 20 allows improving the quality of the monomer solution that is obtained.

Solid and/or liquid impurities R separated from a gas stream G generated by the pyrolysis reactor 4 of the depolymerization installation 2 or the pyrolysis reactor of another depolymerization installation generally contain various molecules, including the monomer (e.g. MMA), oligomers, additives, degradation products, solid residues from the polymer (e.g. PMMA) and other polymers that contaminated the polymer contained in the feed material, also termination by-products.

Returning solid and/or liquid impurities R including the monomer to the pyrolysis reactor 4 allows increasing the yield by recovering the monomer and depolymerized polymer contained in the solid and/or liquid impurities R separated from a gas stream G.

In particular, returning the solid and/or liquid impurities R in a section of the pyrolysis reactor 4 with a limited temperature, in particular a temperature below 300 °C, preferably below 250 °C, preferably below 200 °C appeared more appropriate.

Syrups S are difficult to recycle and the injection of the syrup S in the pyrolysis reactor 4 allows recycling syrups. The syrup S is more efficiently recycled with injection of the syrup in an upstream section of the pyrolysis reactor 4 such that the syrup is subjected to the temperature profile, in particular to the progressively increasing then progressively decreasing temperature.

Unexpectedly, it is possible to depolymerize more feed material when syrup is fed in the pyrolysis reactor 4 than when only feed material is depolymerized.

For a same amount of energy consumed in the pyrolysis reactor 4, a higher total mass flow can be processed when syrup is fed in the pyrolysis reactor 4 in addition to the feed material.

The pyrolysis reactor 4 provided as a heated screw extruder uses mechanical and thermal energy for depolymerizing the polymer.

When injecting the syrup S in the pyrolysis reactor 4, it is expected that the mechanical energy need is reduced.

Unexpectedly, the mechanical energy need remains stable. However, a lot of mechanical energy is provided in the first barrel sections 40 of the barrel 8 and once the polymer is melted, less mechanical energy is needed and more thermal energy is needed.

Hence, the point of injection of the syrup S shall be properly chosen so that, at point of injection of the syrup S, the feed material F is already melted and that the viscosity of the molten polymer has been sufficiently reduced so that most of the mechanical energy has been consumed.

In practice, depending on the length of the barrel 8 and the number of barrels sections 40, this often corresponds to barrels sections 40 comprised between the second barrel section 40 and the sixth barrel section 40 of the series of barrels 40 of the barrel 8.

In the production method, the injection of each the solid and/or liquid impurities and the syrup in the pyrolysis reactor 4 is for example operated continuously, at a constant rate or a varying rate, or intermittently, e.g. with injection phases alternating with non-injection phases. Varying rate and/or intermittent injection allow working with higher rates during high rate phase or injection phases, which may be easier to operate.

Examples 1 to 3 were implemented with injection of a syrup into the pyrolysis reactor 4.

Examples 1 to 3 were implemented in a depolymerization installation 2 comprising the pyrolysis reactor 4, a condenser 6, a feeder 12, a separation unit 14 and a gas cleaning unit 20.

The pyrolysis reactor 4 was provided as a twin-screw extruder comprising a barrel 8 and two parallel screws 10 with co-rotation of the screws 10 and intermeshing of the screws 10. The barrel 8 was made of a series of barrel sections 40. Heaters 42 were provided for generating a temperature profile along the barrel 8, the temperature profile progressively increasing and then decreasing along the barrel 8, with a maximum profile temperature at an intermediate section of the barrel 8.

More specifically, the twin-screw extruder was a TEX44 having a 47mm screw diameter. For industrial production line, a TEX90 having a 96.5mm screw diameter may be used according to the requested feeding rate. TEX extruders are marketed by The Japan Steel Works, Ltd which also offers larger units.

In operation, the pyrolysis reactor 4 had, along the length of the barrel 8, a melting section followed by a depolymerization section. The feed material F was melted in the melting zone of the pyrolysis reactor 4 by shear stress from screws 10 and heat from the heaters 42, then the melted material was transported to the depolymerization section. The depolymerization section of the pyrolysis reactor 4 could reach a temperature of 550°C.

The condenser 6 was provided as a tube and shell heat exchanger.

The feeder 12 was as a gravimetric screw feeder 12.

The separation unit 14 consisted in a main separator 16. The separation unit 14 was not provided with an auxiliary separator 18 located upstream the main separator 16.

The transfer between the separation unit 14 and the condenser 6 was performed via a transfer column 22 having a inner diameter of 5.08 cm and an internal provided as filter mesh. The filter mesh was a cylindrical grid with holes having a diameter of 1 mm. The filter mesh was inserted at an angle in the transfer column 22 so that the gas stream G was filtered through the filter mesh. The filter mesh was placed in the upper part of the transfer column 18, closer to the condenser 6 than to the separation unit 14.

The filter mesh was operated "dry", i.e. without monomer solution being reinjected in the transfer column.

The pyrolysis reactor 4 was configured with a barrel 8 made of a series of seventeen barrel sections named barrel section #1 to barrel section #17 in the following and equipped with an injection port of injection the MMA syrup located at the barrel section #5.

The table 1 below indicates the configuration of the pyrolysis reactor 4, and in particular the function of each barrel section 40 and the set temperature of each barrel section 40 during the implementation of examples 1, 2, 3:

**Table 1:**

| Barrel section # | Function | Heating (°C) | | Barrel section # | Function | Heating (°C) |
|---|---|---|---|---|---|---|
| 1 | Feeding port | 250 | | 10 | | 470 |
| 2 | Inert Gas | 250 | | 11 | | 470 |
| 3 | Melting | 250 | | 12 | | 470 |
| 4 | Plug formation | 250 | | 13 | | 470 |
| 5 | Syrup Injection | 250 | | 14 | | 470 |
| 6 | | 470 | | 15 | | 470 |
| 7 | | 470 | | 16 | Buffer | 250 |
| 8 | | 470 | | 17 | Buffer | 250 |
| 9 | | 470 | | | | |

The barrel sections #16 and #17 are set at 250 °C, but with the heat transfer from the previous barrel section and hot gas, the actual temperature is usually higher.

The feed material used was cast clear PMMA sheets crushed into particles with a size of about 5 to 10 mm.

The syrup was a PMMA syrup containing a white pigment. The syrup was produced several days in advance and was a remaining from a cast sheet production. The syrup had an acceptable viscosity to be pumped into the pyrolysis reactor 4. The syrup was stored in a tank at room temperature and under a controlled atmosphere. The syrup was pumped from the tank to the pyrolysis reactor 4 using a dosing pump that could feed up to 30 kg/h.

The feed material was depolymerized at 470 °C in the pyrolysis reactor 4 with a feed rate increased from 0 to 100 kg/h, and after 25 minutes of operation at 100 kg/h, a first sample of MMA solution was collected (Example 1a).

Then, the feeding rate was reduced to 90 kg/h, and after 30 minutes stabilization a second sample of MMA solution was collected (Example 1b).

Then, the feed rate was reduced to 80 kg/h, and the syrup injection was started at 10 kg/h in the fifth barrel section (barrel section #5). No significant variation in the screw temperature profile was recorded. After stabilization, a sample of MMA solution was collected (Example 2).

After 30 minutes operation, the feed rate was kept at 80 kg/h and the syrup injection was increased to 30 kg/h, and, after stabilization, a new sample of MMA solution was collected. (Example 3).

During the implementation of examples 1 and 2, the temperature in barrel section #6 was of respectively 455 °C and 453 °C. During the implementation of example 3, the temperature in barrel section #6 decreased to 413 °C in example 3. Besides, the temperatures of the barrel sections #7 and #8 were affected by less than 10 °C, which is not significant, and the temperature of the barrel section #9 was not affected.

The samples collected in examples 1a, 1b, 2 and 3 were analyzed by gas chromatography analysis to determine the MMA content of the MMA solution and some key impurities content.

As can be seen in the Table 2 below showing the results of the analysis, the MMA content remained around 95 to 96 wt%. Methanol and methyl isobutyrate content remained in the same range. Methyl acrylate and ethyl acrylate were comonomers present in the Cast PMMA used as feed material. Their content decreased as the PMMA scraps were partly substituted by the syrup which did not contain those comonomers. The reduction in ethyl acrylate from example 1a and 1b to example 2 and 3 follow a linear correlation and is not due to an artifact.

**Table 2**

| SAMPLES | **Example 1a** | **Example 1b** | **Example 2** | **Example 3** |
|---|---|---|---|---|
| Feed rate (kg/h) - CAST / SYRUP | **100/0** | **90/0** | **80/10** | **80/30** |
| Screw rotation (rpm) | 800 rpm | | | |
| Temperature (°C) | 470°C | | | |
| Conditions | 1.0 Bar | | | |
| Crude MMA Color | Clear yellow | Clear yellow | Clear yellow | Clear yellow |
| GC/FID with internal Standard on a polar column | | | | |
| **MMA (wt%) (+/-2%)** | **95** | **95** | **96** | **96** |
| Methanol (wt %) | 0.18 | 0.17 | 0.19 | 0.20 |
| Methyl Acrylate (wt %) | 0.05 | 0.03 | 0.02 | 0.01 |
| Ethyl acrylate (wt%) | 0.52 | 0.49 | 0.39 | 0.27 |
| Methyl isobutyrate (wt%) | 0.08 | 0.08 | 0.08 | 0.07 |
| | | | | |
| GC/FID on apolar column (area%) | | | | |
| unknown heavies (Tr MMA+2min)-(Tr MMA+8min) | 0.32 | 0.34 | 0.32 | 0.29 |
| unknown very heavies > (Tr MMA+8min) | 1.79 | 1.79 | 1.47 | 1.27 |

The amount of heavies in the MMA solution (or "crude MMA") was estimated by the gas chromatography analysis of products having a much higher retention times than the MMA. When substituting part of the PMMA scraps (feed material) by the MMA syrup the amount of heavies determined as gas chromatography peaks area percentage of the products detected, was reduced slightly.

These examples show that it was possible to substitute a significant amount of PMMA scraps with MMA syrup without affecting negatively the crude MMA quality. The heavies did not increase in this case, showing that the MMA oligomers which are present in the syrup did not affect the crude MMA purity.

The productivity of the depolymerization installation is increased. With PMMA scraps only, the maximum productivity of the depolymerization installation was limited to 100 kg/h in the same conditions. But when replacing 20 kg/h of PMMA scraps by MMA syrup, it was possible to feed 30 kg/h of syrup while keeping the performance of the depolymerization installation.

Examples 4, 5, 6 with the pyrolysis reactor 4 configured with the syrup injection port located respectively in barrel sections #12, #15 and #16 instead of barrel #5 may be contemplated.

For such examples 4, 5, 6, the amount of syrup which would be injected limited by the visual detection of a non-depolymerized fraction at the outlet 4B of the pyrolysis reactor 4 or in the separation unit 14. The MMA syrup feeding rate would then be adjusted to the maximum amount, and then the depolymerization installation would be stabilized for data acquisition.

Table 3 below shows expected results for examples 4 to 6.

**Table 3**

| SAMPLES | **Comparative Example 4** | **Comparative Example 5** | **Comparative Example 6** |
|---|---|---|---|
| Feed rate (kg/h) - CAST / SYRUP | **80/10** | **80/5** | **80/3** |
| Liquid injection port / Barrel | **12** | **15** | **16** |
| Screw rotation (rpm) | 800 rpm | | |
| Temperature (°C) | 470 °C | | |
| Conditions | 1.0 Bars | | |

According to these expected results of table 3, the productivity of the depolymerization installation is reduced when the MMA syrup is injected in the downstream half of the barrel 8 and the maximum amount MMA syrup which can be injected in the pyrolysis reactor 4 is limited.

It is supposed that it is preferable to inject the syrup in an upstream section of the barrel 8 of the pyrolysis reactor 4, in particular in a section upstream the section(s) at the maximum profile temperature.

Examples 7 to 12 may be implemented notable with providing a gas cleaning unit 20 and with reinjecting liquid heavies collected in the main separator 16 to the pyrolysis reactor 4. The depolymerization installation 2 would thus be modified as follows:
a) An auxiliary separator 18 is added in the separation unit 14. This auxiliary separator 18 is intended for trapping solid dust and not fully depolymerized PMMA exiting the pyrolysis reactor 4. The auxiliary separator 18 comprises a duct for circulation of the gas stream, the duct has a circular cross-section with an internal diameter of 400 mm, the duct having a horizontal gas inlet section connected to the outlet 4B of the pyrolysis reactor 4 and a horizontal gas outlet section connected to main separator 16, the gas outlet being located 500 mm higher than the gas inlet, the duct having an intermediate section extending vertically upwardly from the gas inlet section to the gas outlet section, a collector being provided at a bottom end of the intermediate section. The auxiliary separator 18 is supposed to lower the gas velocity in the intermediate section such that dust falls to the bottom of the intermediate section and is collected in the collector. The auxiliary separator 18 is intended to be preheated above 100 °C to prevent MMA condensation.
b) The main separator 16 is configured such that the gas flows along a helical path towards a center chamber whereby remaining dust can settle in the helical path. The gas is collected via a collecting tube extending centrally in the chamber and exits the main separator 16. A first demisting grid with holes having a diameter of 8 mm is provided in the helical path and a second demisting grid with holes having a diameter of 3 mm holes is provided in the center chamber around the collecting tube.
c) A gas cleaning unit 20 is added, the gas cleaning unit 20 comprising a vertical transfer column 22 having an inner diameter of 200 mm and two sections located on top of each other. The two sections are filled with a packing material (cylindrical packing of 16 mm Pall rings, wall thickness 0.3 mm, weight of 385 kg/m³, 214 000 pieces/m³, 344 m²/m³, from MTE Group, Boezemweg 5, 3255 MC Oude-Tonge, The Netherlands). The two sections have a total height of two meters of packing.
d) The condenser 6 is modified by adding two grids having holes with a diameter of 2 mm placed one on top of the other with a spacing of 5 cm, the two grids being placed on top of the tubes to distribute the gases over the tubes.
e) A reinjection loop 24 is added for injecting MMA solution from the bottom of the condenser 6 to the upper section 22B of the transfer column 22 at an adjustable rate.
f) The main separator 16 and the transfer column 22 are fluidly connected such that the heavies flowing to the bottom of the transfer column 22 are returned to the main separator 16.
g) A reinjection loop 30 is added for collecting heavies are in the main separator 16 and reinjecting these heavies in the pyrolysis reactor 4.

The following adjustable parameters are expected to have impact the quality of the crude MMA and the yield of the depolymerization:
1) The flow rate of crude MMA returning to the top of the transfer column 22. This may impact the temperature at the entrance of the condenser 6, and the temperature at the top of the transfer column 22, but also at the bottom of the transfer column 22. It may also impact the amount of liquid to be reinjected in the pyrolysis reactor 4.
2) The flowrate of liquid heavies reinjected to the pyrolysis reactor 4.
3) The temperature at the outlet 4B of pyrolysis reactor 4. Providing more heat downstream the injection of the liquid heavies can allow evaporating more of the heavies.

For examples 7, 8 and 9, the feed material would be an injection grade PMMA product, from car tail lights collected at a deconstruction site treating end-of-life vehicles, crushed to small particles. These PMMA scraps generally have a mix of colors (red, black, transparent, orange...) and are also contaminated by other polymers such as polycarbonate and ABS.

The PMMA scraps would be fed at a 50 kg/h rate in the pyrolysis reactor 4 operated with a maximum profile temperature of 470 °C and with a screw rotation speed of 800 rpm, with a back flow of crude MMA from the condenser 6 back to the gas cleaning unit 20 adjusted to about 50 kg/h.

The barrel 8 of the pyrolysis reactor 4 would be equipped with an injection port located at barrel section #16 for injecting the liquid heavies collected from the separation unit 14. The pyrolysis reactor 4 would thus be configured as indicated in table 4 below:

**Table 4**

| Barrel section # | Function | Heating (°C) | | Barrel section # | Function | Heating (°C) |
|---|---|---|---|---|---|---|
| 1 | Feeding port | 250 | | 10 | | 470 |
| 2 | Inert Gas | 250 | | 11 | | 470 |
| 3 | Melting | 250 | | 12 | | 470 |
| 4 | Plug formation | 250 | | 13 | | 470 |
| 5 | | 470 | | 14 | | 470 |
| 6 | | 470 | | 15 | | 250 |
| 7 | | 470 | | 16 | Liquid Injection | 250 |
| 8 | | 470 | | 17 | | 250 |
| 9 | | 470 | | | | |

Expected results are indicated in table 6 below.

**Table 6**

| SAMPLES | **Example 7** | **Example 8** | **Example 9** |
|---|---|---|---|
| PMMA Feed rate (kg/h) | **50** | **50** | **50** |
| Heavies recirculation to the pyrolysis reactor (kg/h) | **0** | **10** | **20** |
| Screw rotation (rpm) | 800 rpm | | |
| Temperature (°C) | 470 °C | | |
| Conditions | 1.0 Bars | 1.0 Bars | |
| | | | |
| GC/FID with internal Standard on a polar column | | | |
| **MMA (wt%) (+/-2%)** | **97** | **98** | |
| Methanol (wt %) | 0.39 | 0.35 | |
| Methyl Acrylate (wt %) | 0.85 | 0.68 | |
| Ethyl acrylate (wt%) | 0.25 | 0.26 | |
| Methyl isobutyrate (wt%) | 0.20 | 0.22 | |

For examples 10, 11, and 12, the liquid injection port would be moved to respectively barrels section #5, #9 and #12. The barrel section preceding the barrel section with the liquid injection would be cooled to 250 °C. The pyrolysis reactor 4 would be reconfigured for each example.

For example, the pyrolysis reactor 4 would configured as indicated in table 5 below for the example 12:

**Table 5**

| Barrel section # | Function | Heating | | Barrel section # | Function | Heating |
|---|---|---|---|---|---|---|
| 1 | Feeding port | 250 | | 10 | | 470 |
| 2 | Inert Gas | 250 | | 11 | | 250 |
| 3 | Melting | 250 | | 12 | Liquid Injection | 250 |
| 4 | Plug formation | 250 | | 13 | | 470 |
| 5 | | 470 | | 14 | | 470 |
| 6 | | 470 | | 15 | | 470 |
| 7 | | 470 | | 16 | Buffer | 250 |
| 8 | | 470 | | 17 | Buffer | 250 |
| 9 | | 470 | | | | |

For examples 10, 11, 12, it is expected that the heavies will affect negatively the quality of the crude MMA which is produced. The amount of impurities such as methyl isobutyrate, methyl acrylate, and ethyl acrylate are expected to increase.

## Claims

1. A method for the production of a monomer from a feed material containing a polymer from which the monomer is a constituent, the production method being implemented in a depolymerization installation (2) comprising a pyrolysis reactor (4) and a condenser (6), the production method comprising the steps of:
- pyrolizing the feed material in the pyrolysis reactor (4) so as to generate a gas stream, the feed material travelling from an inlet of the pyrolysis reactor (4) to an outlet of the pyrolysis reactor (4) with being subjected to heat such as to generate the gas stream at the outlet of the pyrolysis reactor (4); and
- condensing the monomer contained in the gas stream in the condenser (6) such as to obtain liquid monomer;
the production method further comprising feeding the pyrolysis reactor (4) with solid and/or liquid impurities separated from the gas stream generated in the depolymerization installation (2) and/or a gas stream generated in another depolymerization installation and/or with a syrup containing the polymer and/or the monomer and/or oligomers of the monomer.

2. The production method according to claim 1, wherein the solid and/or liquid impurities are fed to the pyrolysis reactor (4) in a section of the pyrolysis reactor (4) that is preferably proximate the outlet of the pyrolysis reactor (4), and in particular closer to the outlet than the inlet.

3. The production method according to claim 2, wherein the intermediate section in which the solid and/or liquid impurities are fed to the pyrolysis reactor is at the temperature below 300°C, preferably below 250°C, still preferably below 200°C and/or at a temperature above the boiling temperature of the monomer and/or above 100°C.

4. The production method according to any one of the preceding claims, wherein the syrup is fed to the pyrolysis reactor (4) in an intermediate section of the pyrolysis reactor (4) that is preferably proximate the inlet of the pyrolysis reactor (4), and in particular closer to the inlet than the outlet.

5. The production method according to claim 4, wherein the intermediate section in which the syrup is fed to the pyrolysis reactor (4) is at the temperature below 450°C, preferably below 350°C, still preferably below 300°C, even more preferably below 250°C.

6. The production method according to any one of the preceding claims, wherein solid polymer is injected in the pyrolysis reactor (4) such as to create a solid plug between the point of injection of the syrup and the inlet of the pyrolysis reactor (4).

7. The production method according to any one of the preceding claims, wherein a temperature in an intermediate section of the pyrolysis reactor (4) located between a point of injection of syrup and a point of injection of solid and liquid impurities is above 250 °C, preferably above 350 °C, in particular above 400 °C.

8. The production method according to any one of the preceding claims, wherein the pyrolysis reactor (4) is a heated screw extruder comprising an elongated barrel (8) defined by a series of barrel sections (40) connected in series and at least one screw (10) extending in the barrel (8) for driving the feed material along the barrel (8), each barrel section (40) being provided with a respective heating device (42) for heating the section of the barrel (8) corresponding to this barrel section (40), the inlet being located in a first barrel section (40) of the series of barrel sections (40) and the outlet being located in a ultimate barrel section (40)of the series of barrel sections (40).

9. The production method according to claim 8, wherein solid and liquid impurities are fed in the antepenultimate barrel, the penultimate barrel and/or the ultimate barrel section (40).

10. The production method according to any one of the preceding claims, comprising a step of separating solid and/or liquid impurities from the gas stream in a separation unit (14) before feeding the gas stream to the condenser (6).

11. The production method according to claim 10, comprising a step of cleaning the gas stream between the separation unit (14) and the condenser (6) using a gas cleaning unit (20) fluidly connected in series between the separating unit (14) and the condenser (6), the gas cleaning unit (20) comprising a transfer column (22) containing internals, the gas cleaning unit being fed with cold monomer in liquid state such that the gas stream flows upwardly in the transfer column (22) and the cold monomer flows downwardly in the transfer column (22) by gravity, heavy contaminants contained in the gas stream condense in liquid state on the internals and flow down by gravity towards the bottom of the transfer column (22) with being then transferred to the separation unit (14).

12. The production method according to claim 11, wherein the cold monomer injected in the transfer column (22) is a fraction of the monomer solution collected from the outlet of the condenser (6).

13. The production method according to any one of the preceding claims, wherein the separation unit (14) comprises a main separator (16) and an auxiliary separator (18) fluidly connected in series.

14. The production method according to any one of the preceding claims, wherein the monomer is methyl methacrylate (MMA) and the polymer is poly (methyl methacrylate) (PMMA).

15. Installation for the production of a monomer from a feed material containing a polymer from which the monomer is a constituent, the installation comprising a pyrolysis reactor (4) and a condenser (6), the installation being configured for the implementation of the production method according to any one of the preceding claims.

## Patentansprüche

1. Verfahren zum Herstellen eines Monomers aus einem Ausgangsmaterial, das ein Polymer enthält, von dem das Monomer ein Bestandteil ist, wobei das Herstellungsverfahren in einer Depolymerisationsanlage (2) durchgeführt wird, umfassend einen Pyrolysereaktor (4) und einen Kondensator (6), wobei das Herstellungsverfahren die folgenden Schritte umfasst:
- Pyrolysieren des Ausgangsmaterials im Pyrolysereaktor (4), um einen Gasstrom zu erzeugen, wobei das Ausgangsmaterial von einem Einlass des Pyrolysereaktors (4) zu einem Auslass des Pyrolysereaktors (4) transportiert wird und dabei Wärme ausgesetzt wird, um den Gasstrom am Auslass des Pyrolysereaktors (4) zu erzeugen; und
- Kondensieren des Monomers, das in dem Gasstrom enthalten ist, im Kondensator (6), um ein flüssiges Monomer zu erhalten;
wobei das Herstellungsverfahren ferner das Beschicken des Pyrolysereaktors (4) mit festen und/oder flüssigen Verunreinigungen, die aus dem Gasstrom, der in der Depolymerisationsanlage (2) erzeugt wurde, abgeschieden wurden, und/oder einem Gasstrom, der in einer anderen Depolymerisationsanlage erzeugt wurde, und/oder mit einem Sirup, der das Polymer und/oder das Monomer und/oder Oligomere des Monomers enthält, umfasst.

2. Herstellungsverfahren nach Anspruch 1, wobei die festen und/oder flüssigen Verunreinigungen dem Pyrolysereaktor (4) in einem Abschnitt des Pyrolysereaktors (4) zugeführt werden, der sich vorzugsweise in der Nähe des Auslasses des Pyrolysereaktors (4) und insbesondere näher am Auslass als am Einlass befindet.

3. Herstellungsverfahren nach Anspruch 2, wobei der Zwischenabschnitt, in dem die festen und/oder flüssigen Verunreinigungen dem Pyrolysereaktor zugeführt werden, eine Temperatur von unter 300 °C, vorzugsweise unter 250 °C, noch bevorzugter unter 200 °C und/oder eine Temperatur über der Siedetemperatur des Monomers und/oder über 100 °C aufweist.

4. Herstellungsverfahren nach einem der vorhergehenden Ansprüche, wobei der Sirup dem Pyrolysereaktor (4) in einem Zwischenabschnitt des Pyrolysereaktors (4) zugeführt wird, der sich vorzugsweise in der Nähe des Einlasses des Pyrolysereaktors (4) und insbesondere näher am Einlass als am Auslass befindet.

5. Herstellungsverfahren nach Anspruch 4, wobei der Zwischenabschnitt, in dem der Sirup dem Pyrolysereaktor (4) zugeführt wird, eine Temperatur von unter 450 °C, vorzugsweise unter 350 °C, bevorzugter unter 300 °C und noch bevorzugter unter 250 °C aufweist.

6. Herstellungsverfahren nach einem der vorhergehenden Ansprüche, bei dem festes Polymer in den Pyrolysereaktor (4) eingespritzt wird, sodass ein fester Pfropfen zwischen dem Einspritzpunkt des Sirups und dem Einlass des Pyrolysereaktors (4) entsteht.

7. Herstellungsverfahren nach einem der vorhergehenden Ansprüche, wobei die Temperatur in einem Zwischenabschnitt des Pyrolysereaktors (4), der sich zwischen dem Einspritzpunkt für den Sirup und dem Einspritzpunkt für die festen und flüssigen Verunreinigungen befindet, über 250 °C, vorzugsweise über 350 °C, insbesondere über 400 °C liegt.

8. Herstellungsverfahren nach einem der vorhergehenden Ansprüche, wobei der Pyrolysereaktor (4) ein beheizter Schneckenextruder ist, der einen länglichen Zylinder (8), der durch eine Reihe von in Reihe geschalteten Zylinderabschnitten (40) definiert ist, und mindestens eine Schnecke (10) umfasst, die sich in dem Zylinder (8) erstreckt, um das Ausgangsmaterial entlang des Zylinders (8) zu transportieren, wobei jeder Zylinderabschnitt (40) mit einer entsprechenden Heizvorrichtung (42) zum Beheizen des Abschnitts des Zylinders (8) versehen ist, der diesem Zylinderabschnitt (40) entspricht, wobei der Einlass in einem ersten Zylinderabschnitt (40) der Reihe von Zylinderabschnitten (40) und der Auslass in einem letzten Zylinderabschnitt (40) der Reihe von Zylinderabschnitten (40) angeordnet ist.

9. Herstellungsverfahren nach Anspruch 8, wobei feste und flüssige Verunreinigungen in den vorvorletzten Zylinder, den vorletzten Zylinder und/oder den letzten Zylinderabschnitt (40) transportiert werden.

10. Herstellungsverfahren nach einem der vorhergehenden Ansprüche, umfassend einen Schritt des Trennens von festen und/oder flüssigen Verunreinigungen aus dem Gasstrom in einer Trenneinheit (14), bevor der Gasstrom dem Kondensator (6) zugeführt wird.

11. Herstellungsverfahren nach Anspruch 10, umfassend einen Schritt des Reinigens des Gasstroms zwischen der Trenneinheit (14) und dem Kondensator (6) unter Verwendung einer Gasreinigungseinheit (20), die strömungstechnisch in Reihe zwischen der Trenneinheit (14) und dem Kondensator (6) geschaltet ist, wobei die Gasreinigungseinheit (20) eine Übertragungssäule (22) mit Einbauten umfasst, die Gasreinigungseinheit mit kaltem Monomer in flüssigem Zustand beschickt wird, sodass der Gasstrom in der Übertragungssäule (22) nach oben strömt und das kalte Monomer durch die Schwerkraft in der Übertragungssäule (22) nach unten fließt, schwere Verunreinigungen, die in dem Gasstrom enthalten sind, in flüssigem Zustand an den Einbauten kondensieren und durch die Schwerkraft nach unten zum Boden der Übertragungssäule (22) fließen und dann zur Trenneinheit (14) geleitet werden.

12. Herstellungsverfahren nach Anspruch 11, wobei das in die Übertragungssäule (22) eingespritzte kalte Monomer eine Fraktion der am Auslass des Kondensators (6) gesammelten Monomerlösung ist.

13. Herstellungsverfahren nach einem der vorhergehenden Ansprüche, wobei die Trenneinheit (14) eine Haupttrennvorrichtung (16) und eine Hilfstrennvorrichtung (18) umfasst, die strömungstechnisch in Reihe geschaltet sind.

14. Herstellungsverfahren nach einem der vorhergehenden Ansprüche, wobei das Monomer Methylmethacrylat (MMA) ist und das Polymer Poly(methylmethacrylat) (PMMA) ist.

15. Anlage zum Herstellen eines Monomers aus einem Ausgangsmaterial, das ein Polymer enthält, von dem das Monomer ein Bestandteil ist, wobei die Anlage einen Pyrolysereaktor (4) und einen Kondensator (6) umfasst, wobei die Anlage für die Durchführung des Herstellungsverfahrens nach einem der vorhergehenden Ansprüche konfiguriert ist.

## Revendications

1. Procédé de production d'un monomère à partir d'une matière première contenant un polymère dont le monomère est un constituant, le procédé de production étant mis en œuvre dans une installation de dépolymérisation (2) comprenant un réacteur de pyrolyse (4) et un condenseur (6), le procédé de production comprenant les étapes suivantes :
- pyrolyse de la matière première dans le réacteur de pyrolyse (4) de manière à générer un flux gazeux, la matière première se déplaçant d'une entrée du réacteur de pyrolyse (4) à une sortie du réacteur de pyrolyse (4) tout en étant soumise à la chaleur de manière à générer le flux gazeux à la sortie du réacteur de pyrolyse (4) ; et
- condenser le monomère contenu dans le flux gazeux dans le condenseur (6) de manière à obtenir un monomère liquide ;
le procédé comprenant en outre l'alimentation du réacteur de pyrolyse (4) avec des impuretés solides et/ou liquides séparées du flux gazeux généré dans l'installation de dépolymérisation (2) et/ou d'un flux gazeux généré dans une autre installation de dépolymérisation et/ou avec un sirop contenant le polymère et/ou le monomère et/ou des oligomères du monomère.

2. Procédé de production selon la revendication 1, dans lequel les impuretés solides et/ou liquides sont introduites dans le réacteur de pyrolyse (4) dans une section du réacteur de pyrolyse (4) qui est de préférence proche de la sortie du réacteur de pyrolyse (4), et en particulier plus proche de la sortie que de l'entrée.

3. Procédé de production selon la revendication 2, dans lequel la section intermédiaire dans laquelle les impuretés solides et/ou liquides sont introduites dans le réacteur de pyrolyse est à une température inférieure à 300 °C, de préférence inférieure à 250 °C, de manière davantage préférée inférieure à 200 °C et/ou à une température supérieure à la température d'ébullition du monomère et/ou supérieure à 100 °C.

4. Procédé de production selon l'une quelconque des revendications précédentes, dans lequel le sirop est introduit dans le réacteur de pyrolyse (4) dans une section intermédiaire du réacteur de pyrolyse (4) qui est de préférence proche de l'entrée du réacteur de pyrolyse (4), et en particulier plus proche de l'entrée que de la sortie.

5. Procédé de production selon la revendication 4, dans lequel la section intermédiaire dans laquelle le sirop est introduit dans le réacteur de pyrolyse (4) est à une température inférieure à 450 °C, de préférence inférieure à 350 °C, de manière davantage préférée inférieure à 300 °C, de manière encore davantage préférée inférieure à 250 °C.

6. Procédé de fabrication selon l'une quelconque des revendications précédentes, dans lequel un polymère solide est injecté dans le réacteur de pyrolyse (4) de manière à créer un bouchon solide entre le point d'injection du sirop et l'entrée du réacteur de pyrolyse (4).

7. Procédé de production selon l'une quelconque des revendications précédentes, dans lequel une température dans une section intermédiaire du réacteur de pyrolyse (4) située entre un point d'injection du sirop et un point d'injection des impuretés solides et liquides est supérieure à 250 °C, de préférence supérieure à 350 °C, en particulier supérieure à 400 °C.

8. Procédé de production selon l'une quelconque des revendications précédentes, dans lequel le réacteur de pyrolyse (4) est une extrudeuse à vis chauffée comprenant un cylindre allongé (8) défini par une série de sections de cylindre (40) reliées en série et au moins une vis (10) s'étendant dans le cylindre (8) pour entraîner la matière première le long du cylindre (8), chaque section de cylindre (40) étant pourvue d'un dispositif de chauffage respectif (42) pour chauffer la section du cylindre (8) correspondant à cette section de cylindre (40), l'entrée étant située dans une première section de cylindre (40) de la série de sections de cylindre (40) et la sortie étant située dans une dernière section de cylindre (40)de la série de sections de cylindre (40).

9. Procédé de production selon la revendication 8, dans lequel les impuretés solides et liquides sont introduites dans l'antépénultième cylindre, l'avant-dernier cylindre et/ou la section ultime du cylindre (40).

10. Procédé de production selon l'une quelconque des revendications précédentes, comprenant une étape de séparation des impuretés solides et/ou liquides du flux gazeux dans une unité de séparation (14) avant d'alimenter le condenseur (6) avec le flux gazeux.

11. Procédé de production selon la revendication 10, comprenant une étape de nettoyage du flux de gaz entre l'unité de séparation (14) et le condenseur (6) en utilisant une unité d'épuration des gaz (20) connectée fluidiquement en série entre l'unité de séparation (14) et le condenseur (6), l'unité d'épuration des gaz (20) comprenant une colonne de transfert (22) contenant des éléments internes, l'unité d'épuration des gaz est alimentée en monomère froid à l'état liquide de sorte que le flux gazeux s'écoule vers le haut dans la colonne de transfert (22) et que le monomère froid s'écoule vers le bas dans la colonne de transfert (22) par gravité, les contaminants lourds contenus dans le flux gazeux se condensent à l'état liquide sur les parties internes et s'écoulent par gravité vers le bas de la colonne de transfert (22) pour être ensuite transférés vers l'unité de séparation (14).

12. Procédé de production selon la revendication 11, dans lequel le monomère froid injecté dans la colonne de transfert (22) est une fraction de la solution de monomère collectée à la sortie du condenseur (6).

13. Procédé de production selon l'une quelconque des revendications précédentes, dans lequel l'unité de séparation (14) comprend un séparateur principal (16) et un séparateur auxiliaire (18) reliés fluidiquement en série.

14. Procédé de production selon l'une quelconque des revendications précédentes, dans lequel le monomère est le méthacrylate de méthyle (MMA) et le polymère est le poly (méthacrylate de méthyle) (PMMA).

15. Installation pour la production d'un monomère à partir d'une matière première contenant un polymère dont le monomère est un constituant, l'installation comprenant un réacteur de pyrolyse (4) et un condenseur (6), l'installation étant configurée pour la mise en oeuvre du procédé de production selon l'une quelconque des revendications précédentes.
